# EUROPEAN PATENT SPECIFICATION

(11) **EP 1 805 456 B1**
(45) Date of publication and mention of the grant of the patent: **21.04.2021**
(21) Application number: 05775780.9
(22) Date of filing: 23.08.2005
(51) Int. Cl.: A61M 16/10, A61M 16/20, A61M 16/06

(54) **OXYGEN ADMINISTRATION APPARATUS**
VORRICHTUNG ZUR SAUERSTOFFVERABREICHUNG
APPAREIL D'ADMINISTRATION D'OXYGENE

(30) Priority: 25.08.2004 GB 0418996
(43) Date of publication of application: 11.07.2007
(73) Proprietor: The BOC Group Limited, The Surrey Research Park Guildford Surrey GU2 7XY (GB)
(72) Inventor: EAST, Philip Charles BOC Limited, West Midlands WV1 2EP (GB)
(74) Representative: Gellner, Bernd
(86) International application number: PCT/GB2005/003281
(87) International publication number: WO 2006/021775

(56) References cited:
- EP-A- 0 574 677
- EP-A- 0 930 454
- EP-A- 1 332 782
- DE-A1- 3 708 146
- DE-A1- 19 957 407
- US-A- 5 024 219
- US-A- 5 477 877
- US-A- 5 927 312
- US-B1- 6 520 176

## Description

This invention relates to oxygen administration apparatus.

A conventional apparatus for administering oxygen to a patient comprises a source of oxygen, an oxygen administration device such as a face mask or a nasal cannula and a flexible plastics oxygen conducting line connectible at one end to the source of oxygen and at another end to the administration device. Such an apparatus is described in, for example, US-A-5 024 219 and US-B1-6 520 176.

Many patients, for example, with chronic lung disease now administer oxygen to themselves at home. Oxygen is conventionally supplied as a compressed gas in cylinders. The cylinders are often heavy and patients find them difficult to handle. Moreover, there are considerable logistical problems in ensuring that a patient at home never runs out of oxygen. As a result it is becoming increasingly more commonplace for a so-called oxygen concentrator to be used instead of a cylinder as the source of oxygen. An oxygen concentrator is a device which separates an oxygen-enriched air stream from a flow of atmospheric air. The separation may be effected by an adsorbent or by suitable semi-permeable membranes. If an absorbent is used, it is typically employed in a plurality of beds. When one bed is adsorbing nitrogen from the air, another bed is being regenerated. In this way a bed with unused adsorptive capacity for nitrogen may be continuously presented to the incoming air, thereby allowing a continuous flow of oxygen-enriched air to be produced. Typically the oxygen concentrator includes a pump or compressor that, in use, maintains a continuous flow of air to the adsorbent or membranes.

Not only do oxygen concentrators avoid the above-mentioned problems associated with compressed oxygen cylinders but they are also safer to use.

Nonetheless, it is vital that neither the patient nor anyone else smoke a cigarette or other smokable material in the vicinity of an oxygen administration device. Otherwise, there is an intolerable risk of a serious fire being caused. Patients are therefore given strict instructions not to smoke when administering oxygen or oxygen-enriched air to themselves and not to permit anyone else to smoke in the immediate vicinity of the oxygen administration device. There is, however, the occasional person who is stupid enough to flout these instructions. When this happens there is an immediate risk of a violent conflagration near the patient's face. Moreover, the plastics tubing used to convey the oxygen to the patient will also catch alight and the fire will spread back along the tubing to the concentrator (or oxygen cylinder) itself. There is therefore a long length of burning plastics which may ignite other combustible material, such as soft furnishings, in the patient's living quarters. Indeed, as the tubing burns back towards the concentrator so its combustion is supported by a continuous flow of oxygen-enriched air. Even if a major or fatal fire is avoided, the concentrator can be seriously damaged.

There is therefore a need for an oxygen administration apparatus with improved safety features.

EP-A- 0 930 454 relates to a safety valve for a domestic fuel gas supply. The safety valve has hooks which lock a trigger member in an open valve position against the bias of a spring. Application of sufficient heat causes the hooks to break and thereby permit the spring to close the valve. DE-A-199 57 407 describes a similar arrangement in which thermal weakening of a support member causes the release of a valve member under the bias of a spring into a valve closing position.

According to the present invention there is provided an oxygen administration apparatus comprising a source of oxygen, an oxygen administration device, a flexible oxygen conducting line connectible at one end to the source of oxygen and another end to the oxygen administration device, characterised by a safety valve in the line, the safety valve comprising a shuttle, a sealing member carried by the shuttle, means biasing the shuttle in a valve-closing direction, and a fusible stop preventing closure of the valve, whereby fusing of the stop allows the biasing means to close the valve, wherein the shuttle is displaceable within a hollow body having a hollow stem complementary to a stem of the shuttle and engageable with the line, the fusible stop engaging the interior of the stem of the body.

In the event of a fire, the stop fuses causing the safety valve to close immediately and hence the supply of oxygen to cease. Further the safety valve itself may act as a fire break protecting the source of oxygen from the fire.

Preferably the shuttle has an elongate hollow stem the distal end of which abuts against the fusible stop.

The body preferably has an internal surface that acts as a seat for the sealing member when the valve is in its closed position. The body is desirably lightweight but crush-resistant. In order to fulfil both these criteria the body may be formed of aluminium or an aluminium-based alloy.

Preferably there is a restricted passage for the flow of oxygen between the shuttle and the body member.

Preferably the biasing means is a spring. A helical compression spring may be used, but other arrangements are possible. For example, a leaf spring may be used.

There are a number of different arrangements which may be used to seat the helical compression spring. For example, the helical compression spring may be seated on a foot member which engages an insert which in turn engages the proximal end of the hollow body.

The insert preferably has an elongate stem engageable with the line.

The fusible stop is preferably of a plastics material having a melting point less than that of the material from which the line is made. The said plastics material may, for example, be an acrylic one or a polyamide.

The oxygen administration device is conveniently a nasal cannula.

An oxygen administration apparatus according to the invention will now be described by the way of example with reference to the accompanying drawings, in which:
Figure 1 is a schema of the apparatus; and
Figure 2 is a side elevation, partly in section of the safety valve forming part of the apparatus shown in Figure 1.

The drawings are not to scale.

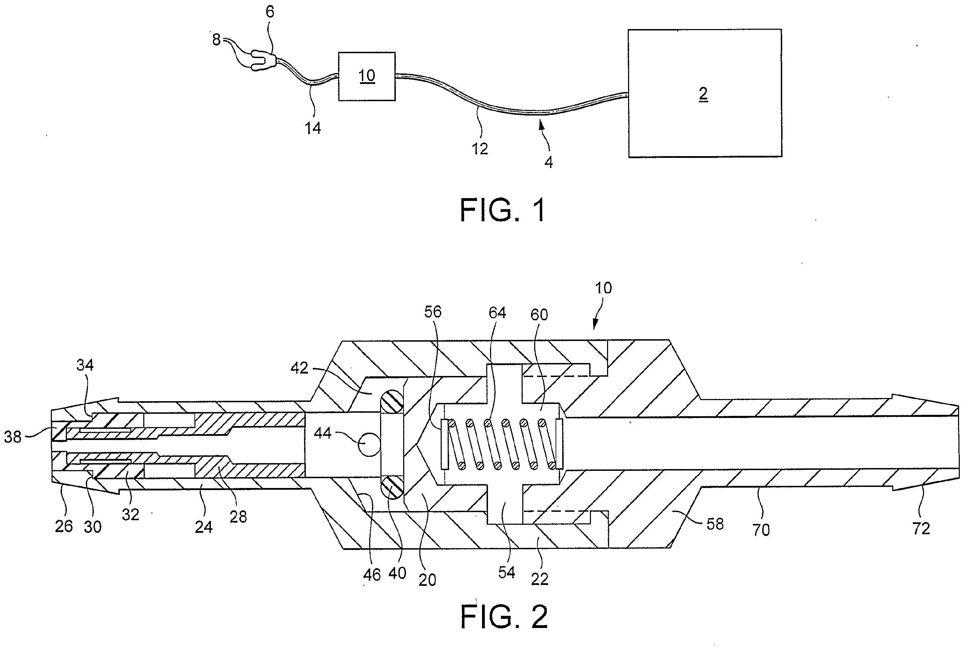

Referring to Figure 1 of the drawings, an oxygen administration apparatus includes an oxygen concentrator 2. An oxygen supply line 4 is attached at one of its end to the oxygen concentrator 2 and at its other end to an oxygen administration device such a nasal cannula 6 having a pair of prongs 8 that the patient can insert in his or her nostrils. A safety valve 10 is located in the oxygen supply line 4 at a region near to the cannula 6.

The oxygen concentrator 2 may be of any standard kind that is used for delivering oxygen-enriched air to a patient. Typically, the oxygen concentrator 2 includes an electrically driven pump (not shown) which delivers a continuous flow of air to separations means (not shown) such as a bed or bed adsorbent for semi-permeable membranes. Typically, the operation of the oxygen concentrator 2 is arranged to supply oxygen-enriched air containing about 28% by volume of oxygen. Such oxygen concentrators are well known in the art and need not be described further herein.

The oxygen supply line 4 typically comprises two lengths 12 and 14 of flexible plastics tubing. The tubing is typically formed of polythene although either plastics material may be used instead. The length 14, which is connected to the cannula 6, is preferably less than one metre in length.

The safety valve 10 is shown in Figure 2. The safety valve 10 comprises a shuttle 20 which is translatable within a hollow body 22. The body 22 has at its distal end an elongate integral stem 24 terminating in an integral nipple 26 to which the length 14 of tubing shown in Figure 1 may be attached. The shuttle 20 has at its distal end an integral hollow elongate stem 28 which is complementary to the stem 24 of the body 22.

The interior wall of the nipple 26 is formed with a shoulder 30. A fusible stop 32 is held in frictional engagement between the stem 24 and the stem 28. The stop 32 takes the form of a plastics sleeve 32. The sleeve 32 has a shoulder 34 complementary to the shoulder 30 of the nipple 26. The engagement of the shoulders 30 and 34 prevents the sleeve 32 from being withdrawn through the nipple in normal use of the safety valve. The distal end of the sleeve 32 is formed with a collar 38 which prevents the stem 28 of the shuttle 20 being withdrawn from the valve through the nipple 26 in normal use.

The stem 28 of the shuttle 20 carries at its proximal end a sealing ring 40. As shown in Figure 2, the safety valve 10 is in its open position and, in operation, in this position, oxygen is able to flow from a chamber 42 defined between the shuttle 20 and the body 22 into a port or ports 44 formed in the wall of the stem 28. In its closed position, however, the sealing ring 40 engages internal surface 46 of the body 22 to form a fluid-tight seal. Further, in this closed position, the port 44 is in such a position downstream of the seal that the oxygen from the chamber 42 does not reach it.

The proximal end of the shuttle 20 is formed with a recess 54. Secured to the shuttle 20 in the recess 54 is a head 56. The proximal end of the body 22 receives the distal end of an elongate insert 58. The insert 58 and the body 22 are in engagement with one another such as they cannot readily be separated. The distal end of the insert 58 has a chamber 60. A foot 62 is located within the chamber 60. One end of a compression spring 64 is seated against the foot 62. The other end of the compression spring 64 bears against the head 56. In the normal open position of the safety valve 10 the compression spring 64 is held under compression and therefore acts against the shuttle 20 in a valve - closing direction. However, the stop in the form of the fusible sleeve 32 prevents translation of the sealing ring 40 from the open position in Figure 2 to its closed position in which it abuts against the inner surface 46 of the body 22.

The insert 58 is formed with an integral elongate hollow stem 70 at its proximal end. The end of the stem of 70 is formed as a nipple 72 to which the length 12 of tubing shown in Figure 1 can be connected.

In operation, oxygen in the form of oxygen-enriched air supplied by the oxygen concentrator flows through the hollow stem 70 of the insert 58. It passes through a restricted passage (not shown) defined between the shuttle 20 and the body 22. Typically, for example, the internal wall of the body 22 may have a hexagonal cross section and the external surface of the shuttle 20 a circular cross-section so as to define gas passages there. The gas flows from these gas passages into the chamber 42. From there the gas flows through the port 44 in the wall of the stem 28 and flows out of the distal end of the valve 10 through the hollow stem 28 of the shuttle 20.

In the event of an oxygen fire occurring near the nasal cannula 6, the length of tubing 14 will typically ignite at its distal end and the fire will rapidly travel backwards to the safety valve 10, oxygen being supplied from the concentrator 2 helping to sustain the resulting conflagration. The fire causes the fusible stop 32 in the form of the sleeve to fuse, thus enabling the compression spring 64 to urge the shuttle 20 forward and carry the sealing ring 40 into valve-closing engagement with the surface 46. Thus, the flow of oxygen-enriched air that supports the conflagration is stopped. Further, the body 22 of the safety valve 10 itself acts as a firebreak. Thus, the fire is prevented from travelling along the oxygen supply line back to the oxygen concentrator 2.

The fusible sleeve 32 is made of a plastics material which has a lower melting point than the length of tubing 14. Typically, if the length of tubing 14 is of polythene, the sleeve 30 may be of a suitable low melting point plastics material which may, for example, be a polyamide (such as ACETAL or NYLON).

The shuttle 20 and the body 22 are preferably made of a suitable lightweight material such as aluminium or an aluminium-based alloy. The wall thickness of the body 22 is preferably such as to impart to it a satisfactory degree of crush resistance so that it will not readily be damaged in normal day-to-day use in a domiciliary environment. The engagement of the body 22 and the insert 58 is preferably such as to make it difficult to dismantle the valve without a custom-made tool for disengaging the insert 58 from the body 20.

## Claims

1. Oxygen administration apparatus comprising a source (2) of oxygen, an oxygen administration device (6), a flexible oxygen-conducting line (4) connectible at one end to the source (12) of oxygen and at another end to the oxygen administration device (6), **characterised by** a safety valve (10) in the line (4), the safety valve (10) comprising a shuttle (20), a sealing member (40) carried by the shuttle (20), means (64) biasing the shuttle (20) in a valve closing direction, and a fusible stop preventing closure of the valve (10), whereby fusing of the stop (32) allows the biasing means (64) to close the valve (10), wherein the shuttle (20) is displaceable within a hollow body (22) having a hollow stem (24) complementary to a stem (28) of the shuttle (20) and engageable with the line (14), the fusible stop (32) engaging the Interior of the stem (24) of the body (22).

2. Oxygen administration apparatus as claimed in claim 1, wherein the shuttle (20) has a hollow stem (28), the distal end of which stem (28) abuts against the fusible stop (32).

3. Oxygen administration apparatus as claimed in claim 1, in which the body (22) has an internal surface (46) that acts as a seat for the sealing member (40) when the valve (10) is in its closed position.

4. Oxygen administration apparatus as claimed in claim 1, 2 or claim 3, in which the body (22) is lightweight but crush-resistant.

5. Oxygen administration apparatus as claimed in any one of claims 1 to 4, including a restricted passage for oxygen between the shuttle (20) and the body (22).

6. Oxygen administration apparatus as claimed in any one of the preceding claims, wherein the biasing means (64) is a spring.

7. Oxygen administration apparatus as claimed in claim 6, wherein the spring is a helical compression spring.

8. Oxygen administration apparatus as claimed in claim 7, wherein the helical compression spring is seated on a foot member (62) which engages an insert (58) which in turn engages the proximal end of the hollow body (22).

9. Oxygen administration apparatus as claimed in claim 8, wherein the insert (58) has a hollow elongate stem (70) engageable with the line (41).

10. Oxygen administration apparatus as claimed in any one of the preceding claims, wherein the fusible stop (32)is of a plastics material having a melting point less than that of the material from which the line is made.

11. Oxygen administration apparatus as claimed in claim 10 in which the said plastics material is an acrylic one.

12. Oxygen administration apparatus as claimed in any one of the preceding claims, wherein the oxygen administration device (6) is a nasal cannula.

13. Oxygen administration apparatus as claimed in any one of the preceding claims, wherein the source (2) of oxygen is an oxygen concentrator.

## Patentansprüche

1. Sauerstoffverabreichungsgerät, umfassend eine Sauerstoffquelle (2), eine Sauerstoffverabreichungsvorrichtung (6), eine flexible sauerstoffführende Leitung (4), die an einem Ende mit der Sauerstoffquelle (12) und an einem anderen Ende mit der Sauerstoffverabreichungsvorrichtung (6) verbindbar ist, **gekennzeichnet durch** ein Sicherheitsventil (10) in der Leitung (4), wobei das Sicherheitsventil (10) ein Schiffchen (20), ein Dichtungselement (40), das von dem Schiffchen (20) getragen wird, Mittel (64), das das Schiffchen (20) in einer Ventilschließrichtung vorspannt, und einen schmelzbaren Anschlag, der das Schließen des Ventils (10) verhindert, umfasst, wobei das Schmelzen des Anschlags (32) dem Vorspannmittel (64) ermöglicht, das Ventil (10) zu schließen, wobei das Schiffchen (20) innerhalb eines hohlen Körpers (22), der einen hohlen Schaft (24) aufweist, der zu einem Schaft (28) des Schiffchens (20) komplementär ist und mit der Leitung (14) in Eingriff bringbar ist, verschiebbar ist, wobei der schmelzbare Anschlag (32) in das Innere des Schafts (24) des Körpers (22) eingreift.

2. Sauerstoffverabreichungsgerät nach Anspruch 1, wobei das Schiffchen (20) einen hohlen Schaft (28) aufweist, wobei das distale Ende des Schafts (28) gegen den schmelzbaren Anschlag (32) anstößt.

3. Sauerstoffverabreichungsgerät nach Anspruch 1, bei dem der Körper (22) eine innere Oberfläche (46) aufweist, die als ein Sitz für das Dichtungselement (40) wirkt, wenn sich das Ventil (10) in seiner geschlossenen Position befindet.

4. Sauerstoffverabreichungsgerät nach Anspruch 1, 2 oder Anspruch 3, bei dem der Körper (22) leichtgewichtig, aber druckbeständig ist.

5. Sauerstoffverabreichungsgerät nach einem der Ansprüche 1 bis 4, das einen eingeschränkten Durchgang für Sauerstoff zwischen dem Schiffchen (20) und dem Körper (22) einschließt.

6. Sauerstoffverabreichungsgerät nach einem der vorstehenden Ansprüche, wobei das Vorspannmittel (64) eine Feder ist.

7. Sauerstoffverabreichungsgerät nach Anspruch 6, wobei die Feder eine Schraubendruckfeder ist.

8. Sauerstoffverabreichungsgerät nach Anspruch 7, wobei die Schraubendruckfeder auf einem Fußelement (62) sitzt, das in einen Einsatz (58) eingreift, der wiederum in das proximale Ende des hohlen Körpers (22) eingreift.

9. Sauerstoffverabreichungsgerät nach Anspruch 8, wobei der Einsatz (58) einen hohlen länglichen Schaft (70) aufweist, der mit der Leitung (41) in Eingriff bringbar ist.

10. Sauerstoffverabreichungsgerät nach einem der vorstehenden Ansprüche, wobei der schmelzbare Anschlag (32) aus einem Kunststoffmaterial besteht, das einen Schmelzpunkt niedriger als der des Materials, aus dem die Leitung hergestellt ist, aufweist.

11. Sauerstoffverabreichungsgerät nach Anspruch 10, bei dem das Kunststoffmaterial ein acrylisches ist.

12. Sauerstoffverabreichungsgerät nach einem der vorstehenden Ansprüche, wobei die Sauerstoffverabreichungsvorrichtung (6) eine Nasenkanüle ist.

13. Sauerstoffverabreichungsgerät nach einem der vorstehenden Ansprüche, wobei die Sauerstoffquelle (2) ein Sauerstoffkonzentrator ist.

## Revendications

1. Appareil d'administration d'oxygène comprenant une source (2) d'oxygène, un dispositif d'administration d'oxygène (6), une ligne flexible conductrice d'oxygène (4) pouvant être raccordée au niveau d'une extrémité à la source (12) d'oxygène et au niveau d'une autre extrémité au dispositif d'administration d'oxygène (6), **caractérisé par** une soupape de sûreté (10) dans la ligne (4), la soupape de sûreté (10) comprenant une navette (20), un élément d'étanchéité (40) transporté par la navette (20), un moyen (64) de sollicitation de la navette (20) dans une direction de fermeture de soupape, et un arrêt fusible empêchant une fermeture de la soupape (10), moyennant quoi une fusion de l'arrêt (32) permet au moyen de sollicitation (64) de fermer la soupape (10), dans lequel la navette (20) est déplaçable à l'intérieur d'un corps creux (22) ayant une tige creuse (24) complémentaire à une tige (28) de la navette (20) et pouvant venir en prise avec la ligne (14), l'arrêt fusible (32) venant en prise avec l'intérieur de la tige (24) du corps (22).

2. Appareil d'administration d'oxygène tel que revendiqué dans la revendication 1, dans lequel la navette (20) a une tige creuse (28), tige (28) dont l'extrémité distale vient en butée contre l'arrêt fusible (32).

3. Appareil d'administration d'oxygène tel que revendiqué dans la revendication 1, dans lequel le corps (22) a une surface interne (46) qui joue le rôle de siège pour l'élément d'étanchéité (40) lorsque la soupape (10) est dans sa position fermée.

4. Appareil d'administration d'oxygène tel que revendiqué dans la revendication 1, 2 ou la revendication 3, dans lequel le corps (22) est léger mais résistant à l'écrasement.

5. Appareil d'administration d'oxygène tel que revendiqué dans l'une quelconque des revendications 1 à 4, incluant un passage restreint pour de l'oxygène entre la navette (20) et le corps (22).

6. Appareil d'administration d'oxygène tel que revendiqué dans l'une quelconque des revendications précédentes, dans lequel le moyen de sollicitation (64) est un ressort.

7. Appareil d'administration d'oxygène tel que revendiqué dans la revendication 6, dans lequel le ressort est un ressort de compression hélicoïdal.

8. Appareil d'administration d'oxygène tel que revendiqué dans la revendication 7, dans lequel le ressort de compression hélicoïdal est monté sur un élément de pied (62) qui vient en prise avec un insert (58) qui à son tour vient en prise avec l'extrémité proximale du corps creux (22).

9. Appareil d'administration d'oxygène tel que revendiqué dans la revendication 8, dans lequel l'insert (58) a une tige allongée creuse (70) pouvant venir en prise avec la ligne (41).

10. Appareil d'administration d'oxygène tel que revendiqué dans l'une quelconque des revendications précédentes, dans lequel l'arrêt fusible (32) est en une matière plastique ayant un point de fusion inférieur à celui du matériau à partir duquel la ligne est fabriquée.

11. Appareil d'administration d'oxygène tel que revendiqué dans la revendication 10 dans lequel ladite matière plastique est une matière acrylique.

12. Appareil d'administration d'oxygène tel que revendiqué dans l'une quelconque des revendications précédentes, dans lequel le dispositif d'administration d'oxygène (6) est une canule nasale.

13. Appareil d'administration d'oxygène tel que revendiqué dans l'une quelconque des revendications précédentes, dans lequel la source (2) d'oxygène est un concentrateur d'oxygène.
